# EUROPEAN PATENT APPLICATION

(11) **EP 3 730 082 A1**
(43) Date of publication of application: **28.10.2020**
(21) Application number: 20171224.7
(22) Date of filing: 24.04.2020
(51) Int. Cl.: A61B 18/14, A61B 90/00

(54) **SINGLE-USE SURGICAL INSTRUMENT**

(30) Priority: 26.04.2019 US 201962838958 P; 18.03.2020 US 202016823024
(71) Applicant: Covidien LP, Mansfield, MA 02048 (US)
(72) Inventor: KUMAR, Sunny, 5000049 Hyderabad (IN); MANDULA, Rajanikanth, 5000040 Hyderabad (IN); MUJAWAR, Arifmohamad, 416301 Sangli, MH (IN)
(74) Representative: Maschio, Antonio

(57) **Abstract**

A single-use surgical instrument includes a break-away assembly configured to break when first and second housing halves of a handle assembly are separated for re-processing. In particular, the break-away assembly is configured to operatively support an actuation handle thereon such that damage to the break-away assembly inhibits proper actuation of the actuation handle, thereby rendering the surgical instrument inoperable.

## Description

### CROSS-REFERENCE TO RELATED APPLICATION

This application claims the benefit of and priority to U.S. Provisional Patent Application No. 62/838,958 filed April 26, 2019, the entire disclosure of which is incorporated by reference herein.

### BACKGROUND

### Technical Field

The disclosure relates generally to the field of surgical instruments. In particular, the disclosure relates to single-use surgical instruments including a breakable housing inhibiting re-processing thereof.

### Background of Related Art

Instruments such as electrosurgical forceps are commonly used in open and minimally invasive surgical procedures to coagulate, cauterize and seal tissue. Such forceps typically include a pair of jaw members that can be controlled by a surgeon to grasp targeted tissue, such as, e.g., a blood vessel. The jaw members may be approximated to apply a mechanical clamping force to the tissue, and are associated with at least one electrode to permit the delivery of electrosurgical energy to the tissue. The combination of the mechanical clamping force and the electrosurgical energy has been demonstrated to join adjacent layers of tissue captured between the jaw members. When the adjacent layers of tissue include the walls of a blood vessel, sealing the tissue may result in hemostasis, which may facilitate the transection of the sealed tissue.

A bipolar electrosurgical forceps typically includes opposed electrodes disposed on clamping faces of the jaw members. The electrodes are charged to opposite electrical potentials such that an electrosurgical current may be selectively transferred through tissue grasped between the electrodes. To affect a proper seal, particularly in relatively large vessels, the pressure applied to the vessel and the gap distance established between the electrodes are controlled.

Such instruments may be single-use instruments that are prepackaged as sterile. Such single-use instruments ease the logistical burden and help to ensure that medical professionals are using sterile instruments in their best condition.

### SUMMARY

In accordance with an embodiment of the disclosure, a handle assembly for use with a surgical instrument includes first and second housing halves, a movable handle movably coupled to at least one of the first or second housing halves, and a break-away assembly. The break-away assembly includes a weakened portion on the at least one of the first or second housing halves, and a first coupling member including first and second joints coupled to the respective first and second housing halves. The first joint is coupled to the weakened portion. The first and second joints are inter-locked such that separation of the first and second housing halves breaks the weakened portion to inhibit actuation of the movable handle.

In an embodiment, the movable handle may be pivotably secured to the weakened portion.

In another embodiment, the first coupling member may be disposed adjacent the movable handle.

In yet another embodiment, the weakened portion may have a thickness of about 0.02 inch.

In still yet another embodiment, the first and second joints and the respective first and second housing halves may be formed as a single construct.

In still yet another embodiment, the first and second joints and the respective first and second housing halves may be monolithically formed.

In an embodiment, the first and second housing halves may be ultrasonically welded.

In another embodiment, the first joint of the first coupling member may define a cavity, and the second joint of the first coupling member may include a tooth configured to be securely received in the cavity.

In yet another embodiment, the second joint of the first coupling member may define a slot configured to receive at least a portion of the first joint therein.

In still yet another embodiment, the break-away assembly may further include a second coupling member including third and fourth joints coupled to the respective first and second housing halves. The third joint may be coupled to the weakened portion. The third and fourth joints may be inter-locked such that separation of the first and second housing halves breaks the weakened portion to inhibit actuation of the movable handle.

In an embodiment, at least a portion of the movable handle may be interposed between the first and second coupling members.

In yet another embodiment, the first and second coupling members may define longitudinal axes that are orthogonal to each other.

In still yet another embodiment, the first and second coupling members may be adjacent the pivot of the movable handle.

In an embodiment, at least one of the first or second coupling members may include a snap-fit configuration.

In accordance with another embodiment of the disclosure, a single-use surgical instrument includes a handle assembly, an elongate member extending distally from the handle assembly, and an end effector supported on the elongate member and operatively coupled to the handle assembly. The handle assembly includes first and second housing halves, a movable handle pivotably coupled to at least one of the first or second housing halves about a pivot, and a break-away assembly. The break-away assembly includes a weakened portion on the at least one of the first or second housing halves and a first coupling member including first and second joints coupled to the respective first and second housing halves. The first joint coupled to the weakened portion. The first and second joints are coupled to each other such that separation of the first and second housing halves breaks the weakened portion to inhibit actuation of the movable handle about the pivot.

In an embodiment, the first coupling member may be substantially parallel to a longitudinal axis defined by the elongate member.

In accordance with yet another embodiment of the disclosure, a single-use handle assembly includes first and second housing halves, a movable handle, and a break-away assembly. The first housing half has a first portion having a first thickness and a second portion having a second thickness less than the first thickness. The movable handle is movably coupled to the second portion of the first housing. The break-away assembly includes a first coupling member including first and second snap joints coupled to the respective first and second housing halves. The first snap joint is coupled to the second portion of the first housing half. The first and second snap joints are inter-locked such that separation of the first and second housing halves breaks the second portion of the first housing to inhibit actuation of the movable handle.

### BRIEF DESCRIPTION OF THE DRAWINGS

Various embodiments of the disclosure are described hereinbelow with reference to the drawings, wherein:
FIG. 1 is a perspective view of a single-use surgical instrument in accordance with an embodiment of the disclosure;
FIG. 2 is a top perspective view of a handle assembly of the single-use surgical instrument of FIG. 1 with a first housing half removed;
FIG. 3 is a bottom perspective view of the handle assembly of the single-use surgical instrument of FIG. 1 with a second housing half removed; and
FIGS. 4-6 are partial perspective views of the handle assembly of the single-use surgical instrument of FIG. 1 with portions of the first and second housing halves removed.

### DETAILED DESCRIPTION

Embodiments of the disclosure will now be described in detail with reference to the drawings, in which like reference numerals designate identical or corresponding elements in each of the several views. As used herein, the term "distal," as is conventional, will refer to that portion of the instrument, apparatus, device or component thereof which is farther from the user while, the term "proximal," will refer to that portion of the instrument, apparatus, device or component thereof which is closer to the user. In the following description, well-known functions or constructions are not described in detail to avoid obscuring the disclosure in unnecessary detail.

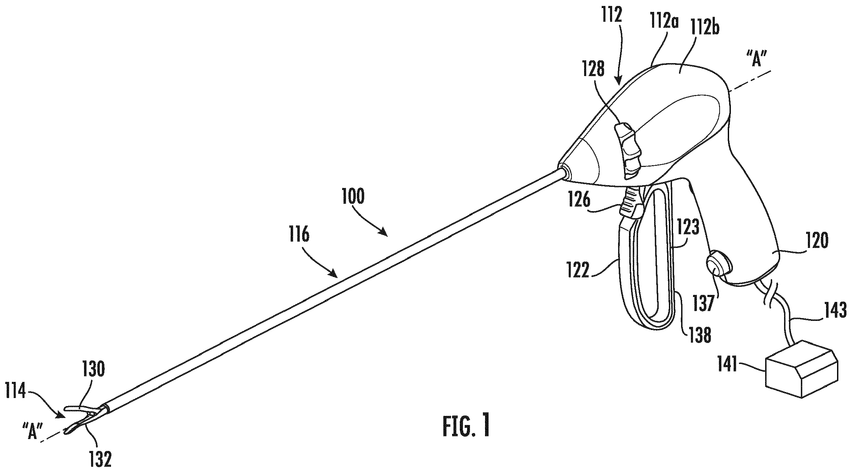

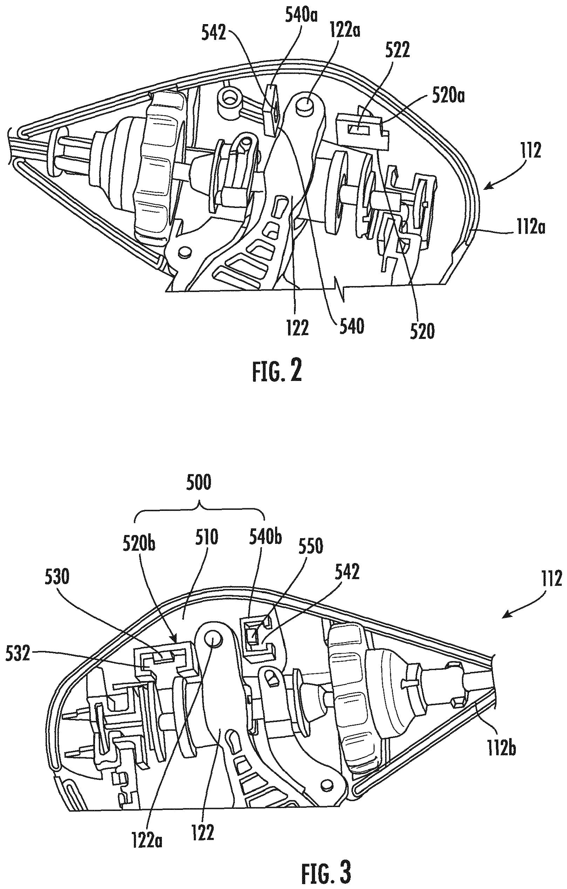

FIG. 1 illustrates a surgical instrument 100 generally including a handle assembly 112 that supports various actuators thereon for remotely controlling an end effector 114 through an elongated shaft 116. The surgical instrument 100 is a single-use device that is discarded after use. In particular, the handle assembly 112 includes a break-away assembly 500 (FIG. 3) in accordance with an embodiment of the disclosure to inhibit reprocessing and re-use of the single-use surgical instrument 100. Although this configuration is typically associated with instruments for use in laparoscopic or endoscopic surgical procedures, various aspects of the disclosure may be practiced with traditional open instruments and in connection with endoluminal procedures as well.

The handle assembly 112 is constructed of a first housing half 112a and a second housing half 112b. The first and second housing halves 112a, 112b may be joined by, e.g., adhesives, ultrasonic welding, or other suitable assembly methods. The handle assembly 112 has a stationary handle 120, a movable handle 122, a trigger 126, and a rotation knob 128. The movable handle 122 is operable to move the end effector 114 between an open configuration wherein a pair of opposed upper and lower jaw members 130, 132 are disposed in spaced relation to one another, and a closed or clamping configuration wherein the jaw members 130, 132 are closer together. Approximation of the movable handle 122 with the stationary handle 120 moves the end effector 114 to the closed configuration, and separation of the movable handle 122 from the stationary handle 120 transitions the end effector 114 to the open configuration. The trigger 126 is operable to extend and retract a knife blade (not shown) through the end effector 114 when the end effector 114 is in the closed configuration. The rotation knob 128 rotates the elongated shaft 116 and the end effector 114 about a longitudinal axis "A-A" defined through the elongated shaft 116.

To electrically control the end effector 114, the stationary handle 120 supports a first switch 137 thereon, such as, for example, a depressible button, which is operable by the user to initiate and terminate the delivery of electrosurgical energy to the end effector 114. The first switch 137 is engageable by a button activation post 138 extending from a proximal side 123 of the movable handle 122 upon proximal movement of the movable handle 122 to an actuated or approximated position. The first switch 137 is in electrical communication with an electrosurgical generator 141 via suitable electrical wiring (not explicitly referenced) extending from the housing 112 through a cable 143 extending between the housing 112 and the electrosurgical generator 141. The generator 141 may include devices such as the LigaSure® Vessel Sealing Generator and the ForceTriad® Generator sold by Covidien. The cable 143 may include a connector (not shown) thereon such that the surgical instrument may be selectively coupled electrically to the generator 141.

FIGS. 2 and 3 illustrate the first and second housing halves 112a, 112b of the handle assembly 112 including the break-away assembly 500 in accordance with an embodiment of the disclosure. The break-away assembly 500 is configured to inhibit reprocessing and re-use of the surgical instrument 100 by breaking or damaging the handle assembly 112 when the first and second housing halves 112a, 112b are separated for reprocessing, thereby rendering the single-use surgical instrument 100 inoperable. In an embodiment, the break-away assembly 500 is disposed adjacent the movable handle 122 pivotably supported on the handle assembly 112 about a pivot 122a. In particular, the break-away assembly 500 includes a weakened portion 510 on the second housing half 112b. The weakened portion 510 pivotably supports the movable handle 122 thereon. For example, the pivot 122a may be disposed on the weakened portion 510. In order to facilitate damage to the housing assembly 112 to render the surgical instrument 100 inoperable, the weakened portion 510 may have a reduced thickness to promote damage when the first and second housing halves 112a, 112b are separated. For example, the weakened portion 510 may include a thickness of about 0.02 inch. In an embodiment, the thickness may be less than about 0.02 inch. Alternatively, the weakened portion 510 may be formed of a fragile or brittle material.

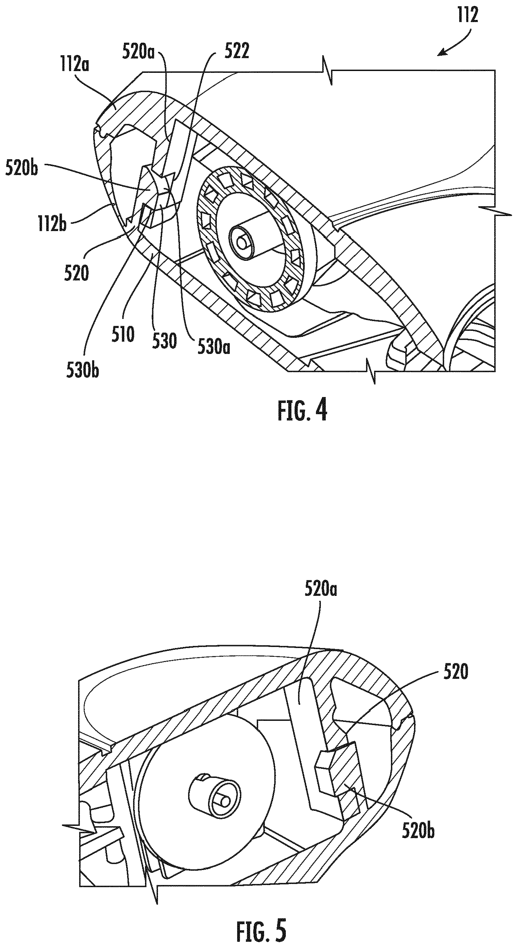

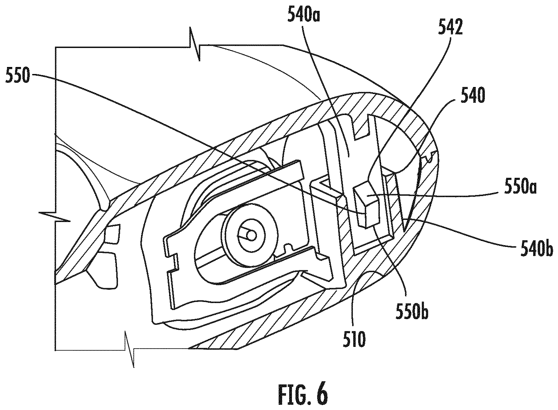

The break-away assembly 500 further includes first and second coupling members 520 (FIG. 4), 540 (FIG. 6) secured to the weakened portion 510. The pivot 122a of the movable handle 122 may be interposed between the first and second coupling members 520, 540.

The first coupling member 520 includes a first joint 520a secured to the first housing half 112a, and a second joint 520b secured to the second housing half 112b. The first joint 520a and the first housing half 112a may be formed as a single construct, and similarly the second joint 520b and the second housing half 112b may be formed as a single construct. For example, the first joint 520a and the first housing half 112a may be monolithically formed, and similarly the second joint 520b and the second housing half 112b may be monolithically formed. In addition, the first and second joints 520a, 520b may include a snap-fit configuration. In particular, the first joint 520a protrudes inwardly from the first housing half 112a and defines a cavity 522. The first joint 520a may have, e.g., a rectangular profile, extending substantially parallel to the longitudinal axis "A-A" (FIG. 1) of the elongated shaft 116.

The second joint 520b protrudes inwardly from the second housing half 112b. The second joint 520b defines a slot 532 having a configuration complementary to that of the first joint 520a to receive at least a portion of the first joint 520a therein. For example, the second joint 520b may have, e.g., a C-shaped or a U-shaped profile. For example, a length of the second joint 520b may extend substantially parallel to the longitudinal axis "A-A" of the elongated shaft 116. In addition, the second joint 520b further includes a tooth 530 extending into the slot 532 such that when the first joint 520a is received in the slot 532 of the second joint 520, the tooth 530 is configured to be securely received in the cavity 522 of the first joint 520a to form an inter-locking structure.

The second coupling member 540 may have a similar configuration as the first coupling member 520. The second coupling member 540 includes the first joint 540a coupled to the first housing half 112a, and the second joint 540b coupled to the weakened portion 510 of the second housing half 112b. The first joint 540a and the first housing half 112a may be formed as a single construct, and the second joint 540b and the second housing half 112b may be formed as a single construct. For example, the first and second joints 540a, 540b and the respective first and second housing halves 112a, 112b may be monolithically formed. The first joint 540a protrudes inwardly from the first housing half 112a, and defines a cavity 542. The first joint 540a may have, e.g., a rectangular, profile extending substantially orthogonal to the longitudinal axis "A-A" of the elongated shaft 116. The second joint 540b protrudes inwardly from the second housing half 112b. The second joint 540b defines a slot 542 dimensioned to receive at least a portion of the first joint 540a. For example, the second joint 540b has a configuration complementary to that of the first joint 540 such as, e.g., a C-shaped or a U-shaped profile. For example, a length of the second joint 540b extends substantially orthogonal to the longitudinal axis "X-X." In addition, the second joint 540b further includes a tooth 550 extending into the slot 542 such that when the first joint 540a is received in the slot 542 of the second joint 540b, the tooth 550 is configured to be securely received in the cavity 542 of the first joint 540a when the first and second joints 540a, 540b are mated. Under such a configuration, the first and second coupling members 520, 540 are substantially orthogonal to each other such that various forces applied to the handle assembly 112 during separation of the first and second housing halves 112a, 112b ensure damage to the weakened portion 510 to render the surgical instrument 100 inoperable, i.e., by failing to properly support the movable handle 122.

FIGS. 4-6 illustrate the handle assembly 112 in an assembled state, in which, the first and second housing halves 112a, 112b are interlocked by the first and second coupling members 520, 540 such that separation of the first and second housing halves 112a, 112b breaks the weakened portion 510 to render the surgical instrument 100 inoperable, as explained above. In particular, FIGS. 4 and 5 illustrate the first joint 520a of the first coupling member 520 mated with the second joint 520b. The tooth 530 of the second joint 520b has a slanted portion 530a configured to facilitate receipt of the tooth 530 in the cavity 522 of the first joint 520a. The tooth 530 further includes an engaging portion 530b in planar contact with the cavity 522. Under such a configuration, the tooth 530 may travel in a single direction towards the cavity 522 to be mated. However, once the tooth 530 is placed within the cavity 522, the tooth 530 is inter-locked with the cavity 522 and securely remains within the cavity 522. In this manner, the first and second joints 520a, 520b operate as a single construct after the first and second housing halves 112a, 112b are assembled.

FIG. 6 illustrates the second coupling member 540 in an assembled state. In particular, the first joint 540a is mated with the second joint 540b to form an inter-locking structure. The tooth 550 of the second joint 540b has a slanted portion 550a configured to facilitate insertion of the tooth 550 in the cavity 542 of the first joint 540a. The tooth 550 further includes an engaging portion 550b in planar contact with the cavity 542. Under such a configuration, the tooth 550 may travel in a single direction towards the cavity 542 to be mated. However, once the tooth 550 is placed within the cavity 542, the tooth 550 remains secured within the cavity 542. In this manner, the first and second joints 540a, 540b operate as a single construct after the first and second housing halves 112a, 112b are assembled such that separation of the first and second housing halves 112a, 112b breaks the weakened portion 510 to render the handle assembly 112 inoperable, as previously discussed.

While the embodiment illustrates the weakened portion 510 disposed in the second housing half 112b, it is contemplated that the weakened portion 510 may be placed in both of the first and second housing halves 112a, 112b and coupled with the respective first joints 520a, 540a and second joints 520b, 540b. Alternatively, the weakened portion 510 may be placed only in the first housing half 112a. While the single-use surgical instrument 100 is shown to include electrosurgical forceps, it is contemplated that the single-use surgical instrument 100 may be configured to be used with other tool assemblies.

In use, a clinician may wish to clamp and seal tissue between the jaw members 130, 132 of the end effector 114. To move the end effector 114 from the open configuration to the closed configuration, the movable handle 122 is moved generally proximally from a spaced position toward the approximated position. The movable handle 122 is moved to the fully approximated position, whereby the activation post 138 of the movable handle 122 contacts the first switch 137. Upon actuating the first switch 137 with the activation post 138, electrosurgical energy is transmitted from the electrosurgical generator 141 to the end effector 114 to seal the tissue disposed between the jaw members 130, 132. After the procedure, the single-use surgical instrument 100 is discarded. However, any attempts to re-process the surgical instrument 100 in order to re-use the single-use surgical instrument 100 renders the surgical instrument 100 inoperable. As discussed hereinabove, separation of the first and second housing halves 112a, 112b for re-processing damages the weakened portion 510 which inhibits proper actuation of the movable handle 122.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as examples of particular embodiments. Those skilled in the art will envision other modifications within the scope and spirit of the claims appended hereto.

Although the foregoing disclosure has been described in some detail by way of illustration and example, for purposes of clarity or understanding, it will be obvious that certain changes and modifications may be practiced within the scope of the appended claims.

The invention may be described by reference to the following numbered paragraphs:-
1. A handle assembly for use with a surgical instrument comprising:
   first and second housing halves;
   a movable handle movably coupled to at least one of the first or second housing halves;
   a break-away assembly including:
      a weakened portion on the at least one of the first or second housing halves; and
      a first coupling member including first and second joints coupled to the respective first and second housing halves, the first joint coupled to the weakened portion, wherein the first and second joints are inter-locked such that separation of the first and second housing halves breaks the weakened portion to inhibit actuation of the movable handle.
2. The handle assembly according to paragraph 1, wherein the movable handle is pivotably secured to the weakened portion.
3. The handle assembly according to paragraph 1, wherein the first coupling member is disposed adjacent the movable handle.
4. The handle assembly according to paragraph 1, wherein the weakened portion has a thickness of about 0.02 inch.
5. The handle assembly according to paragraph 1, wherein the first and second joints and the respective first and second housing halves are formed as a single construct.
6. The handle assembly according to paragraph 1, wherein the first and second joints and the respective first and second housing halves are monolithically formed.
7. The handle assembly according to paragraph 1, wherein the first and second housing halves are ultrasonically welded.
8. The handle assembly according to paragraph 1, wherein the first joint of the first coupling member defines a cavity, and the second joint of the first coupling member includes a tooth configured to be securely received in the cavity.
9. The handle assembly according to paragraph 1, wherein the second joint of the first coupling member defines a slot configured to receive at least a portion of the first joint therein.
10. The handle assembly according to paragraph 1, wherein the break-away assembly further includes a second coupling member including third and fourth joints coupled to the respective first and second housing halves, the third joint coupled to the weakened portion, the third and fourth joints are inter-locked such that separation of the first and second housing halves breaks the weakened portion to inhibit actuation of the movable handle.
11. The handle assembly according to paragraph 10, wherein at least a portion of the movable handle is interposed between the first and second coupling members.
12. The handle assembly according to paragraph 10, wherein the first and second coupling members define longitudinal axes that are orthogonal to each other.
13. The handle assembly according to paragraph 10, wherein the first and second coupling members are adjacent the pivot of the movable handle.
14. The handle assembly according to paragraph 10, wherein at least one of the first or second coupling members includes a snap-fit configuration.
15. A single-use surgical instrument comprising:
   a handle assembly including:
      first and second housing halves;
      a movable handle pivotably coupled to at least one of the first or second housing halves about a pivot;
      a break-away assembly including:
         a weakened portion on the at least one of the first or second housing halves; and
         a first coupling member including first and second joints coupled to the respective first and second housing halves, the first joint coupled to the weakened portion, wherein the first and second joints are coupled to each other such that separation of the first and second housing halves breaks the weakened portion to inhibit actuation of the movable handle about the pivot;
   an elongate member extending distally from the handle assembly; and
   an end effector supported on the elongate member and operatively coupled to the handle assembly.
16. The single-use surgical instrument according to paragraph 15, wherein the first and second joints of the first coupling member have a snap-fit configuration.
17. The single-use surgical instrument according to paragraph 15, wherein the first coupling member is substantially parallel to a longitudinal axis defined by the elongate member.
18. The single-use surgical instrument according to paragraph 15, wherein the second joint defines a slot configured to receive at least a portion of the first joint therein.
19. The single-use surgical instrument according to paragraph 15, wherein the first coupling member is disposed adjacent the pivot.
20. A single-use handle assembly comprising:
   first and second housing halves, the first housing half having a first portion having a first thickness and a second portion having a second thickness less than the first thickness;
   a movable handle movably coupled to the second portion of the first housing;
   a break-away assembly including:
      a first coupling member including first and second snap joints coupled to the respective first and second housing halves, the first snap joint coupled to the second portion of the first housing half, wherein the first and second snap joints are inter-locked such that separation of the first and second housing halves breaks the second portion of the first housing to inhibit actuation of the movable handle.

## Claims

1. A handle assembly for use with a surgical instrument comprising:
first and second housing halves;
a movable handle movably coupled to at least one of the first or second housing halves;
a break-away assembly including:
a weakened portion on the at least one of the first or second housing halves; and
a first coupling member including first and second joints coupled to the respective first and second housing halves, the first joint coupled to the weakened portion, wherein the first and second joints are inter-locked such that separation of the first and second housing halves breaks the weakened portion to inhibit actuation of the movable handle.

2. The handle assembly according to claim 1, wherein the movable handle is pivotably secured to the weakened portion.

3. The handle assembly according to claim 1 or claim 2, wherein the first coupling member is disposed adjacent the movable handle.

4. The handle assembly according to any preceding claim, wherein the weakened portion has a thickness of about 0.02 inch; and/or wherein the first and second joints and the respective first and second housing halves are formed as a single construct; and/or wherein the first and second joints and the respective first and second housing halves are monolithically formed; and/or wherein the first and second housing halves are ultrasonically welded.

5. The handle assembly according to any preceding claim, wherein the first joint of the first coupling member defines a cavity, and the second joint of the first coupling member includes a tooth configured to be securely received in the cavity; and/or wherein the second joint of the first coupling member defines a slot configured to receive at least a portion of the first joint therein.

6. The handle assembly according to any preceding claim, wherein the break-away assembly further includes a second coupling member including third and fourth joints coupled to the respective first and second housing halves, the third joint coupled to the weakened portion, the third and fourth joints are inter-locked such that separation of the first and second housing halves breaks the weakened portion to inhibit actuation of the movable handle; preferably wherein at least a portion of the movable handle is interposed between the first and second coupling members.

7. The handle assembly according to claim 6, wherein the first and second coupling members define longitudinal axes that are orthogonal to each other; and/or wherein the first and second coupling members are adjacent the pivot of the movable handle.

8. The handle assembly according to claim 6 or claim 7, wherein at least one of the first or second coupling members includes a snap-fit configuration.

9. A single-use surgical instrument comprising:
a handle assembly including:
first and second housing halves;
a movable handle pivotably coupled to at least one of the first or second housing halves about a pivot;
a break-away assembly including:
a weakened portion on the at least one of the first or second housing halves; and
a first coupling member including first and second joints coupled to the respective first and second housing halves, the first joint coupled to the weakened portion, wherein the first and second joints are coupled to each other such that separation of the first and second housing halves breaks the weakened portion to inhibit actuation of the movable handle about the pivot;
an elongate member extending distally from the handle assembly; and
an end effector supported on the elongate member and operatively coupled to the handle assembly.

10. The single-use surgical instrument according to claim 9, wherein the first and second joints of the first coupling member have a snap-fit configuration.

11. The single-use surgical instrument according to claim 9 or claim 10, wherein the first coupling member is substantially parallel to a longitudinal axis defined by the elongate member.

12. The single-use surgical instrument according to any of claims 9 to 11, wherein the second joint defines a slot configured to receive at least a portion of the first joint therein.

13. The single-use surgical instrument according to claim 11, wherein the first coupling member is disposed adjacent the pivot.

14. A single-use handle assembly comprising:
first and second housing halves, the first housing half having a first portion having a first thickness and a second portion having a second thickness less than the first thickness;
a movable handle movably coupled to the second portion of the first housing;
a break-away assembly including:
a first coupling member including first and second snap joints coupled to the respective first and second housing halves, the first snap joint coupled to the second portion of the first housing half, wherein the first and second snap joints are inter-locked such that separation of the first and second housing halves breaks the second portion of the first housing to inhibit actuation of the movable handle.
